# EUROPEAN PATENT SPECIFICATION

(11) **EP 4 108 205 B1**
(45) Date of publication and mention of the grant of the patent: **19.11.2025**
(21) Application number: 22180853.8
(22) Date of filing: 23.06.2022
(51) Int. Cl.: A61B 34/30, A61B 90/90, A61B 34/32, A61B 90/94, A61B 90/98, A61B 34/20

(54) **INSTRUMENT IDENTIFICATION FOR SURGICAL ROBOT**
INSTRUMENTENIDENTIFIZIERUNG FÜR EINEN CHIRURGISCHEN ROBOTER
IDENTIFICATION D'INSTRUMENT POUR ROBOT CHIRURGICAL

(30) Priority: 24.06.2021 US 202163214342 P
(43) Date of publication of application: 28.12.2022
(73) Proprietor: Medtech S.A., 34000 Montpellier (FR)
(72) Inventor: BONARIC, Patrice, 34680 St Georges d'orques (FR); COISEUR, Florian, 34970 Lattes (FR)
(74) Representative: Taor, Simon Edward William

(56) References cited:
- EP-A2- 2 135 577
- WO-A1-2019/079212
- WO-A1-2019/139841
- WO-A1-2020/210621
- US-A1- 2017 360 520

## Description

### TECHNICAL FIELD

This document pertains generally, but not by way of limitation, to devices, systems and methods for robot-assisted surgical procedures, such those involving the use of articulating arms that can hold and manipulate instruments. More specifically, but not by way of limitation, the present application relates to instrument and/or instrument holder identification with robotic surgical devices, systems and methods.

### BACKGROUND

Robotic surgical arms have been developed and are used to hold and manipulate various instruments during a procedure. It is desirable to precisely mount instruments to the robotic surgical arm. During a surgery it is common for several instruments and accessories (such as an interface block, interface instrument, holder, guide, adaptor, etc.) to be utilized and changed onto and off the robotic surgical arm in a sequence.

### OVERVIEW

The present inventors recognize that robotic surgical systems, methods and apparatuses can be susceptible to human error and to certain operational errors. For example, such systems, methods and apparatuses may require personnel to properly select and properly install robotically manipulated instruments and associated accessories on the surgical arm during robotic surgical procedures at different stages of the surgery. The robotic surgical arm can utilize one or more accessories specifically designed for a specific instrument. As such, selection of the proper accessory or accessories for a specific instrument can be prone to selection error.

Additionally, robotic surgical systems and techniques can rely on calibration data to facilitate proper operation. This data can be derived by assembling the components (e.g., the instrument and one or more accessories). These components all have dimensional variations in accordance with manufacturing standards. Thus assembled, these components have no repeatable dimensions, and to compensate for this and to guarantee accuracy during the procedure, the components must be calibrated for each stage in the surgery (i.e., every time a new instrument and/or a new one or more accessories are utilized). The calibration data can then processed by an on-board computer and software of the robot.

The present inventors recognize robotic surgical systems, methods and apparatuses that can address human error (improper component selection, improper instrument and/or accessory mounting, improper instrument and/or accessory positioning, improper combination of instrument and accessory, etc.) and/or certain operational errors such as corrupted instrument calibration data, and certain software malfunctions. As discussed further herein, identification apparatuses, systems and methods are utilized with such robotic surgical systems, methods and apparatuses. These identification systems can be used to ensure that personnel select the correct instrument and/or one or more accessories. This can include alerting personnel to a counterfeit instrument or accessory. The identification systems can be used to ensure that personnel have properly mounted and/or positioned the instrument and/or one or more accessories on the robotic surgical arm. Additionally, identification systems can ensure accurate calibration data are loaded for the robotic surgical system. This can ensure application accuracy. The identification systems can also facilitate a faster more user-friendly user interface, reduce operation time and reduce stress related to human error. WO2019/079212, EP2135577 and US2017/360520 disclose prior art relevant for the present invention.

This overview is intended to provide an overview of subject matter of the present patent application. It is not intended to provide an exclusive or exhaustive explanation of the invention which is defined by the appended independent claims. The detailed description is included to provide further information about the present patent application.

### BRIEF DESCRIPTION OF THE DRAWINGS

FIG. 1 is a diagrammatic view of an operating room including a robot-assisted surgical system comprising a robotic arm, a computing system and an identification system according to an example of the present application.
FIG. 2 is an enlarged view of an end of the robotic arm of FIG. 1 showing an end effector, an accessory, and an instrument and other components according to an example of the present application.
FIG. 3 is a schematic view of the base and the robotic arm of FIG. 2 additionally including a first data system including a data reader for identifying various medical instruments and/or accessories during surgeries performed with the surgical robot, such with the robot-assisted surgical system of FIGS. 1 and 2.
FIGS. 4A and 4B are a perspective views of the instrument with a data system according to an example of the present application.
FIG. 4C is an enlargement of indicia on the instrument of FIGS. 4A and 4C.
FIG. 5 is a perspective view of the instrument with a second data system according to another example of present application.
FIGS. 6A and 6B are perspective views of a third data system according to another example of the present application.
FIG. 7 is a flow chart illustrating steps of a first method of validating one or more of an instrument or accessory for use during a robotically performed surgical procedure according to an example of the present application.
FIG. 8 is a flow chart illustrating steps of a second method of validating one or more of an instrument or accessory for use during a robotically performed surgical procedure according to an example of the present application.
FIG. 9 is a flow chart illustrating steps of a third method of validating one or more of an instrument or accessory for use during a robotically performed surgical procedure according to an example of the present application.
FIG. 10 is a flow chart illustrating steps of a fourth method of validating one or more of an instrument or accessory for use during a robotically performed surgical procedure according to an example of the present application.
FIG. 11 is a schematic illustration of a robotic surgical system incorporating an identification system of the present application interacting with other systems .
FIG. 12 is a schematic illustration of a block diagram of an example machine upon which any one or more of the techniques discussed herein may perform and with which any of the devices discussed herein may be used in accordance with some examples.

In the drawings, which are not necessarily drawn to scale, like numerals may describe similar components in different views. Like numerals having different letter suffixes may represent different instances of similar components. The drawings illustrate generally, by way of example, but not by way of limitation, various embodiments discussed in the present document.

### DETAILED DESCRIPTION

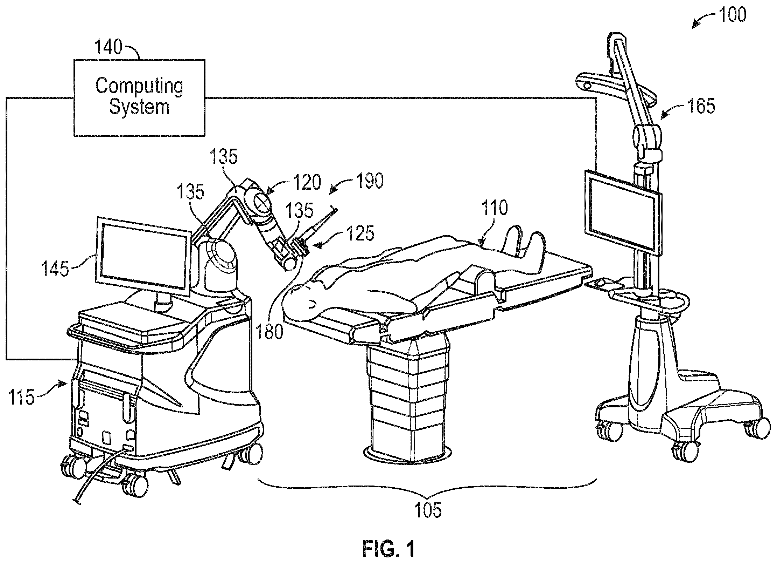

FIG. 1 illustrates a surgical system 100 for operation in surgical area 105 of a patient 110 in accordance with at least one example of the present disclosure. Surgical area 105 in one example can include an anatomy of the patient 110. Surgical area 105 can include any surgical area of the patient 110, including but not limited to the skull, shoulder, hip, knee, elbow, thumb, spine, and the like. Surgical system 100 can also include a robotic system 115 with one or more robotic arms, such as a robotic arm 120. As illustrated, the robotic system 115 can utilize only a single robotic arm. The robotic arm 120 can be a 6 degree-of-freedom (DOF) robot arm, such as the ROSA^{®} robot from Medtech, a Zimmer Biomet Holdings, Inc. company. In some examples, the robotic arm 120 can be cooperatively controlled with surgeon input on the end effector or surgical instrument, such as surgical instrument 125. In other examples, the robotic arm 120 can operate autonomously. While not illustrated in FIG. 1, one or more positionable surgical support arms can be incorporated into surgical system 100 to assist in positioning and stabilizing instruments or anatomy during various procedures.

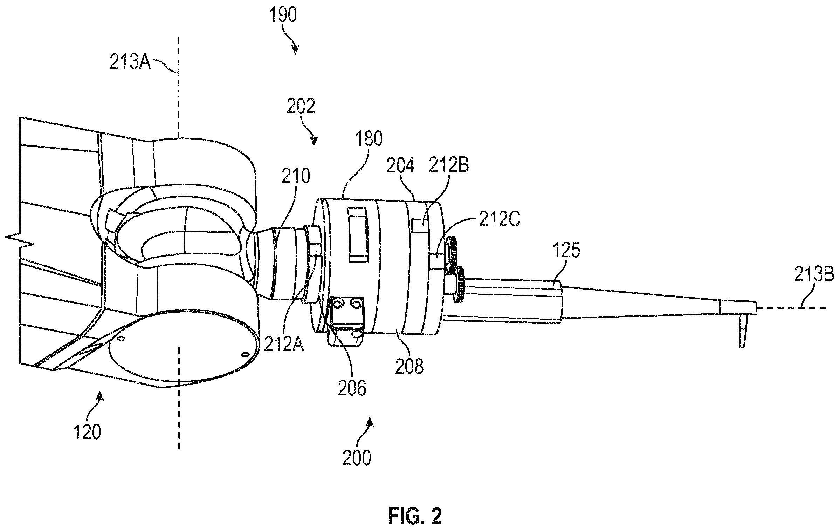

Each robotic arm 120 can rotate axially and radially and can receive a surgical instrument, or end effector, 125 at distal end. The surgical instrument 125 can be any surgical instrument adapted for use by the robotic system 115, including, for example, a guide tube, a holder device, a gripping device such as a pincer grip, a burring device, a reaming device, an impactor device such as a humeral head impactor, a pointer, a probe or the like. In certain examples, the pointer can be a laser pointer that can generate a laser beam or array that is used for alignment of implants during surgical procedures. The surgical instrument 125 can be positionable by the robotic arm 120, which can include multiple robotic joints, such as joints 135, that allow surgical instrument 125 to be positioned at any desired location adjacent or within a given surgical area 105. As discussed below, the robotic arm 120 can be used with an instrument positioning device, e.g., an instrument holder 200 (FIG. 2), to position an instrument in a known orientation relative to the surgical area 105 based on a virtual coordinate system such as one determined by computing system 140.

The computing system 140 can be part of the surgical system 100. The computing system 140 can operate the robotic arm 120 and the surgical instrument 125 according to some examples. The computing system 140 can include at least memory, a processing unit or processing circuitry, and user input devices, as will be described herein. Computing system 140 can also include a human interface device 145 for providing images for a surgeon to be used during surgery. The computing system 140 is illustrated as a separate standalone system, but in some examples the computing system 140 can be integrated into the robotic system 115. The human interface device 145 can provide images, including but not limited to three-dimensional images of bones, glenoid, joints, and the like of the patient. The human interface device 145 can include associated input mechanisms, such as a touch screen, foot pedals, or other input devices compatible with a surgical environment.

The computing system 140 can receive pre-operative medical images of a relevant anatomy of the patient. These images can be received in any manner and the images can include, but are not limited to, computed tomography (CT) scans, magnetic resonance imaging (MRI), two-dimensional x-rays, three-dimensional x-rays, ultrasound, and the like. These images in one example can be sent via a server as files attached to an email. In another example the images can be stored on an external memory device such as a memory stick and coupled to a USB port of the robotic system to be uploaded into the processing unit. In yet other examples, the images can be accessed over a network by computing system 140 from a remote storage device or service.

After receiving one or more images, the computing system 140 can generate one or more virtual models related to the surgical area 105. Alternatively, the computing system 140 can receive virtual models of the anatomy of the patient prepared remotely. Specifically, a virtual model of the anatomy of the patient 110 can be created by defining anatomical points within the image(s) and/or by fitting a statistical anatomical model to the image data. The virtual model, along with virtual representations of implants, can be used for calculations related to the desired height, depth, inclination angle, or version angle of an implant, stem, surgical instrument, or the like related to be utilized in the surgical area 105. In another procedure type, the virtual model can be utilized to determine insertion location, trajectory and depth for inserting an instrument. The virtual model can also be used to determine bone dimensions, implant dimensions, bone fragment dimensions, bone fragment arrangements, and the like. Any model generated, including three-dimensional models, can be displayed on human interface device 145 for reference during a surgery or used by the robotic system 115 to determine motions, actions, and operations of the robotic arm 120 or the surgical instrument 125. Known techniques for creating virtual bone models can be utilized, such as those discussed in U.S. Patent No. 9,675,461, titled "Deformable articulating templates" or U.S. Patent No. 8,884,618, titled "Method of generating a patient-specific bone shell" both by Mohamed Rashwan Mahfouz, as well as other techniques known in the art.

The computing system 140 can also communicate with the tracking system 165 that can be operated by the computing system 140 as a stand-alone unit. The surgical system 100 can utilize the Polaris optical tracking system from Northern Digital, Inc. of Waterloo, Ontario, Canada. Additionally, the tracking system 165 can comprise the tracking system shown and described in Pub. No. US 2017/0312035, titled "Surgical System Having Assisted Navigation" to Brian M. May. The tracking system 165 can monitor a plurality of tracking elements, affixed to objects of interest to track locations of multiple objects within the surgical field. The tracking system 165 functions to create a virtual three-dimensional coordinate system within the surgical field for tracking patient anatomy, surgical instruments, or portions of the robotic system 115. The tracking elements can be tracking frames including multiple IR reflective tracking spheres, or similar optically tracked marker devices. In one example, tracking elements can be placed on or adjacent one or more bones of patient 110. In other examples, tracking elements can be placed on the robotic arm 120, the surgical instrument 125, and/or an the implant to accurately track positions within the virtual coordinate system associated with the surgical system 100. In each instance the tracking elements can provide position data, such as patient position, bone position, joint position, robotic arm position, implant position, or the like.

The robotic system 115 can include various additional sensors and guide devices. For example, the robotic system 115 can include one or more force sensors, such as a force sensor 180. The force sensor 180 can provide force data or information to the computing system 140 of the robotic system 115. The force sensor 180 can be used to monitor various forces during certain operations, sense instrument and/or accessory weight, etc. Monitoring forces can assist in preventing negative outcomes through force fitting components. In other examples, the force sensor 180 can provide additionally information such as on soft-tissue tension in the tissues surrounding a target joint.

In order to ensure that the computing system 140 is moving the robotic arm 120 in a known and fixed relationship to the surgical area 105 and the patient 110, the space of the surgical area 105 and the patient 110 can be registered to the computing system 140 via a registration process involving registering fiducial markers attached to patient 110 with corresponding images of the markers in patient 110 recorded preoperatively or just prior to a surgical procedure. For example, a plurality of fiducial markers can be attached to or in proximity to the patient 110, images of the patient 110 with the fiducial markers can be taken or obtained and stored within a memory device of the computing system 140. Subsequently, the patient 110 with the fiducial markers can be moved into, if not already there because of the imaging, the surgical area 105 and the robotic arm 120 can touch each of the fiducial markers. Engagement of each of the fiducial markers can be cross-referenced with, or registered to, the location of the same fiducial marker in the images. As such, the real-world, three-dimensional geometry of the anatomy attached to the fiducial markers can be correlated to the anatomy in the images and movements of the instruments 125 attached to the robotic arm 120 based on the images will correspondingly occur in the surgical area 105.

Subsequently, other instruments and accessory devices can be attached to the surgical system 100. These can be positioned by the robotic arm 120 into a known and desired orientation relative to the anatomy, these can be calibrated, and can be utilized in steps in the surgery. As discussed previously, such attachment and removal of instruments and accessories can be subject to human and other types of error. Thus, the present surgical system 100 can include an identification system 190. Such identification system 190 can ensure the use of proper surgical instruments and/or accessories corresponding to one another and corresponding to a proper step in the surgery. Identification system 190 can additionally include data regarding usage, safety and/or authentication of the surgical instrument 125 and/or accessories as further discussed herein.

The identification system 190 can use the computing system 140 of the present application or other processing circuitry including cloud based processing circuitry, for example. As further discussed herein, the identification system 190 can include a data storage device, a data reading system and other components as will be discussed herein in further detail subsequently.

FIG. 2 shows an exemplary example of an end of the robotic arm 120 configured as an instrument holder 200 for retaining the surgical instrument 125 according to one example. The instrument holder 200 can include an end effector 202 and an interface instrument 204. The end effector 202 can include the force sensor 180, a flange 206, a interface accessory 208 and a manipulation mechanism 210. Portions of the identification system 190 are also shown in FIG. 2 including a plurality of data storage devices 212A, 212B and 212C.

The end effector 202 can be manipulatable along one or more axes such as via the manipulation mechanism 210 relative to the remainder of the robotic arm 120. The end effector 202 can comprise a stack of components, these components can be selectively removable from the stack and can be configured for use together, for example. The flange 206 can be configured to couple the force sensor 180 to the manipulation mechanism 210. The flange 206 can be removable from the manipulation mechanism 210 in some examples. The force sensor 180 and/or interface accessory 208 can be specifically configured for use with the particular surgical instrument 125 and/or interface instrument 204. As such, the flange 206 or another component such as the force sensor 180 and/or the interface accessory 208 can be provided with the data storage device 212A mounted to or embedded therein. This data storage device 212A can be used to identify the flange 206, force sensor 180 and/or interface accessory 208 (e.g., provide one or more part numbers or other identification). This can be used to identify the flange 206, force sensor 180 and/or interface accessory 208 appropriate for use with the surgical instrument 125 and/or interface instrument 204 during a particular stage in the robotic surgery. Other data storage devices (not shown) can specifically relate to various of these components including identifying only the force sensor 180 or for identifying only the interface accessory 208, for example.

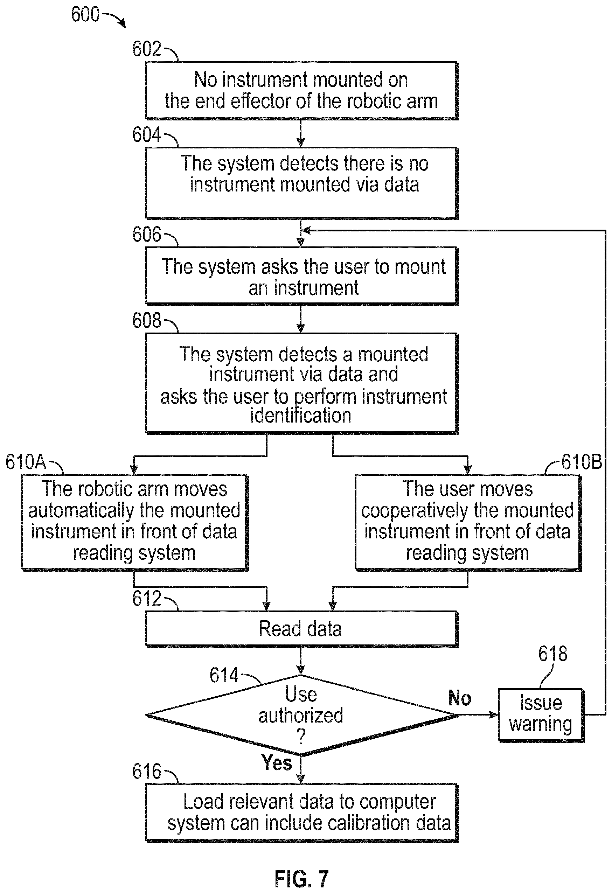

The manipulation mechanism 210 can be movable about and/or along various axes such as the axis 213A and the axis 213B, for example. This, in turn, can manipulate the surgical instrument 125. The surgical instrument 125 can be manipulatable independent of the manipulation mechanism 210. The force sensor 180 can be coupled to the flange 206. The force sensor 180 can provide force data and/or other sensed data to the identification system 190 and/or the computing system 140 of the robotic system 115 (FIG. 1). The force sensor 180 can be used to monitor force, torque, acceleration, vector and/or other criteria during certain operations. The force sensor 180 can be configured to sense the instrument holder and/or instrument weight. This sensed weight can be sent to the identification system 190 and/or the computing system 140 (FIG. 1) as an initial identification data, for example, as further discussed herein in reference to FIGS. 7-9. As different instruments, associated accessories and/or end effectors can have different weights from one another, weight data can be utilized to identify if the instrument holder 200 and/or the surgical instrument 125 have a weight expected for the instrument holder and/or the surgical instrument selected for the associated surgical step(s). Weight data can identify if a surgical instrument is mounted to the instrument holder 200. According to some examples, the force sensor 180 can be configured to provide torque, vector, acceleration or other data that can identify improper mounting between components of the tool stack including an improperly mounted surgical instrument 125 on the interface instrument 204, an improperly mounted interface instrument 204 on the end effector 202, and/or improperly mounted components of the end effector 202, or the like.

The force sensor 180 and/or the data storage device 212A can provide use, safety and/or authentication data to the identification system 190 and/or the computing system 140 (FIG. 1) according to some examples. Such data can include identification data (e.g., a validation, an authentication, a use authorization, an identifier, part number(s)), calibration data, usage data (e.g., number of times used, hours of operation, etc.), sterilization data (number of times sterilized, hours or time sterilized, temperature), operation data (e.g., pressure, force, torque, etc.) and/or other relevant data regarding the end effector 202. Some of such data can be collected by the force sensor 180 according to some examples. Data can be collected by the data storage device 212A, other sensors and/or the computing system 140 (FIG. 1) and can be written to the data storage device 212A after or during the surgery, for example. Such data can also be stored in memory of the computing system 140 (FIG. 1).

The interface accessory 208 can be configured to couple to the force sensor 180. The interface accessory 208 can provide an interface for the interface instrument 204 to mount to. The interface instrument 204 can be and accessory specifically configured for use with the particular surgical instrument 125. The data storage device 212B can be mounted to or embedded in the interface instrument 204. The data storage device 212B can be configured to identify the interface instrument 204 (e.g., provide identification such as a part number) to better ensure the proper accessory to be used with the surgical instrument 125, for example.

The data storage device 212B can provide use, safety and/or authentication data to the identification system 190 and/or the computing system 140 (FIG. 1) according to some examples. Such data can include identification data (e.g., a validation, an authentication, a use authorization, an identifier, part number(s)), calibration data, usage data (e.g., number of times used, hours of operation, etc.), sterilization data (number of times sterilized, hours or time sterilized, temperature), operation data (e.g., pressure, force, torque, etc.) and/or other relevant data regarding the interface instrument 204. Data can be collected by the data storage device 212B, other sensors and/or the computing system 140 (FIG. 1) and can be written to the data storage device 212B after or during the surgery, for example. Such data can also be stored in memory of the computing system 140 (FIG. 1).

The surgical instrument 125 can be configured to mount on the interface instrument 204. As discussed, the surgical instrument 125 can be designed to be used with the particular interface instrument 204 and/or the particular end effector 202 (e.g., a particular force sensor 180). The surgical instrument 125 is illustrated as a pointer probe configured to register particular anatomical points during brain surgery in FIG. 2. However, the illustrated probe is purely exemplary and other surgical instruments are contemplated as part of the systems and methods discussed herein. Indeed, as discussed, multiple instruments can be utilized during the various stages/steps of the robotic surgery.

As shown in FIG. 2, the data storage device 212C can be mounted to or embedded in the interface instrument 204. The data storage device 212C can be configured to identify the surgical instrument 125 (e.g., provide an identifier such as a part number or authentication number) to better ensure the proper surgical instrument is being used, for example.

The data storage device 212C can provide use, safety and/or authentication data to the identification system 190 and/or the computing system 140 (FIG. 1) according to some examples. Such data can include identification data (e.g., a validation, an authentication, a use authorization, an identifier, part number(s)), calibration data, usage data (e.g., number of times used, hours of operation, etc.), sterilization data (number of times sterilized, hours or time sterilized, temperature), operation data (e.g., pressure, force, torque, etc.) and/or other relevant data regarding the surgical instrument 125. Data can be collected by the data storage device 212C, other sensors and/or the computing system 140 (FIG. 1) and can be written to the data storage device 212C after or during the surgery, for example. Such data can also be stored in memory of the computing system 140 (FIG. 1).

It is further contemplated that several data storage devices can be placed on or embedded in a single unique component (e.g., one of the force sensor 180, interface accessory 208, the particular surgical instrument 125 and/or interface instrument 204). For example, one of the several data storage devices can include manufacturing data, another can include operation data, a third could include sterilization data, etc. This could better preserve integrity of data when performing tasks such as rewriting. Furthermore, various types and modalities of data storage devices could be combined on the single unique component.

The data storage devices 212A, 212B, and/or 212C can comprise any data storage device as known in the art. Thus, one or more of the data storage devices 212A, 212B, and/or 212C can comprise optical character devices configured for optical character recognition (OCR). As such these devices can be indicia that is optically identifiable such as a part number, a barcode, Quick Response (QR) code, a data matrix code or the like. One or more of the data storage devices 212A, 212B, and/or 212C can comprise a near field storage device (e.g., a Radio Frequency Identification (RFID) tag, Near Field Communication (NFC) interface (i.e., tag), or the like). One or more of the data storage devices 212A, 212B, and/or 212C can comprise a device configured for use with an electrical contact reading device. In such example, instrument and/or accessory data can be stored by a micro-chip embedded in the surgical instrument 125 and/or the accessory. This data can then be transferred to a data reading device via an electric contact/connection between the surgical instrument 125 and/or accessory and the data reading device. The data storage devices 212A, 212B, and/or 212C can have data writing capability in addition to data reading capability according to some examples as further discussed herein. Data writing can facilitate writing data regarding identification data, calibration data, usage data, sterilization data, operation data and/or other relevant data to one or more of the data storage devices 212A, 212B, and/or 212C during or after the surgical procedure.

According to some examples, the data storage devices 212A, 212B, and/or 212C can have identification data that is linked or otherwise associated according to criteria of the identification system 190 and/or the computing system 140 (FIG. 1). According to this criteria, each of the data storage devices 212A, 212B, and/or 212C must present identification (including authentication, validation, use authorization, part number or other identifier) that matches or is otherwise linked to or validated for use with others of the data storage devices 212A, 212B, and/or 212C. In this manner it can be ensured that only a proper instrument for the surgical stage, proper accessory(s) associated with that instrument and/or a proper end effector associated with that instrument and/or accessory(s) can satisfy all identification data criteria in order to be used during the step of the surgery.

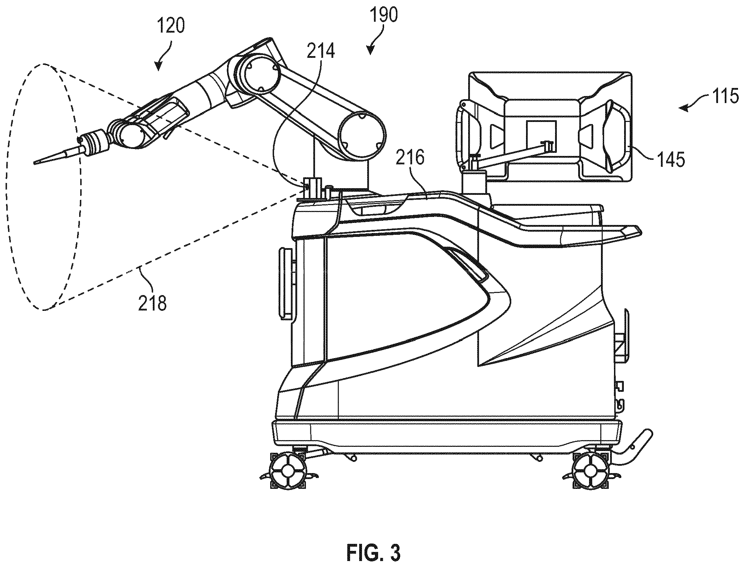

FIG. 3 shows part of the robotic system 115 including the robotic arm 120 and the human interface device 145 (e.g., a display). The identification system 190 can include a data reading device 214 configured to read data from one or more of the data storage devices 212A, 212B, and/or 212C (FIG. 2). The data reading device 214 can be a fixed unit (e.g., part of a base 216 of the robot) and can be mounted to the base 216 adjacent the robotic arm 120. The data reading device 214 can have a range or field 218 sufficient such that the data storage devices 212A, 212B, and/or 212C mounted to the robotic arm 120 fall within the range or field so as to be identifiable for reading and/or writing data thereto. The robotic arm 120 may need to be manipulated to fall within this range or field.

The data reading device 214 can alternatively be a wand or other mobile unit capable of movement (removal) from the base 216 by personnel or can be a device mounted to the robotic arm 120 or another item as further discussed herein. The data reading device can be an optical recognition device, a near field reading device, an electrical contact reading device, or the like.

As shown in FIG. 3, the data reading device 214 can read data from a communication unit that is part of a tag of the RFID or NFC device. The data reading device 214 can send the read data to the computing system 140 (FIG. 1) or other processing circuitry including the cloud via a communications network and identification system 190.

In some examples, the data reading device 214 can utilize a wireless network interface, such as a Wi-Fi interface, Bluetooth interface, cellular data network interface (e.g., a 5G or a 4G LTE interface), and/or another type of wireless network interface. In such examples, the data reading device 214 can use the wireless network interface to send and/or receive data from the computing system 140. In some examples, data reading device 214 can use a communication unit that is a wire-based communication interface, such as a Universal Serial Bus (USB) interface or another type of interface. In such examples, the data reading device 214 can use the wire-based communications interface to send and/or receive data from the computing system 140 (FIG. 1) or other processing circuitry. For instance, the data reading device 214 can use a USB connection with another device, such as a mobile device, that is configured to communicate with the computing system 140 (FIG. 1) or other processing circuitry. In this example, the data reading device 214 can communicate with the computing system 140 (FIG. 1) or other processing circuitry while connected to the mobile device. In some examples, data reading device 214 can utilize an internal communication bus, such as a serial peripheral interface (SPI) bus or I2C bus. In such examples, the data reading device 214 can use the internal communication bus to send and/or receive data from the computing system 140 (FIG. 1) or other processing circuitry.

According to further examples, one or more of the data storage devices 212A, 212B, and/or 212C can utilize the wireless network interface directly without communicating via the data reading device 214. In such examples, one or more of the data storage devices 212A, 212B, and/or 212C can use the wireless network interface to send and/or receive data from the computing system 140 (FIG. 1) or other processing circuitry. In some examples, one or more of the data storage devices 212A, 212B, and/or 212C can use a communication unit that is a wire-based communication interface, such as (USB) interface or another type of interface. In such examples, one or more of the data storage devices 212A, 212B, and/or 212C can use the wire-based communication interface or power connection based communications interface (e.g., electrical contact reading system) to send and/or receive data from the computing system 140 (FIG. 1) or other processing circuitry. For example, the one or more of the data storage devices 212A, 212B, and/or 212C can use a USB connection or other connection with a mobile device, that is configured to communicate with the computing system 140 (FIG. 1) or other processing circuitry. In this example, one or more of the data storage devices 212A, 212B, and/or 212C can communicate with the computing system 140 (FIG. 1) or other processing circuitry while connected to the mobile device. In some examples, one or more of the data storage devices 212A, 212B, and/or 212C can utilize an internal communication bus, such as a serial peripheral interface (SPI) bus or I2C bus. In such examples, one or more of the data storage devices 212A, 212B, and/or 212C can use the internal communication bus to send and/or receive data from the computing system 140 (FIG. 1) or other processing circuitry.

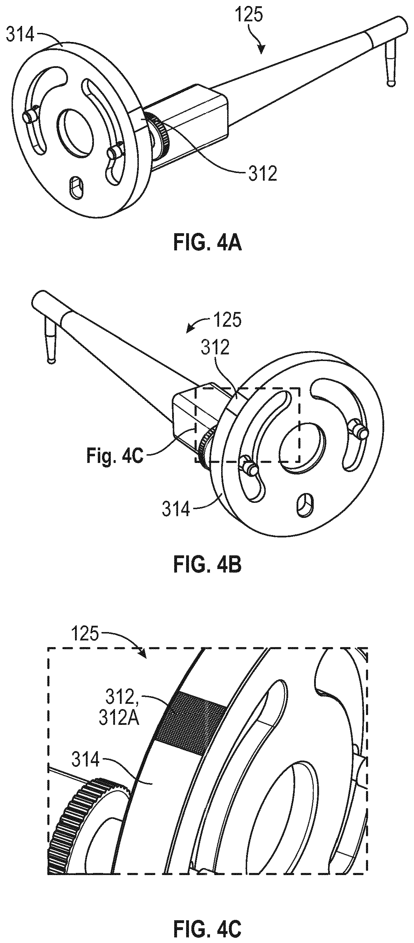

FIGS. 4A-4C show an example of the surgical instrument 125 with a data storage device 312 that can be utilized with the various systems previously discussed. The data storage device 312 can be the near field storage device (e.g., RFID, NFC, etc.) or the optical character device configured for OCR such as a data matrix 312A (FIG. 4C). The data storage device 312 can be mounted, embedded, engraved or otherwise coupled to an exterior surface 314 of the surgical instrument 125. In this location, the data matrix 312A can be visible to a data reading device (e.g., the data reading device 214 of FIG. 3). Several data matrices including the data matrix 312A can be utilized in multiple locations on the exterior surface 314 of the surgical instrument 125 as shown in FIGS. 4A and 4B.

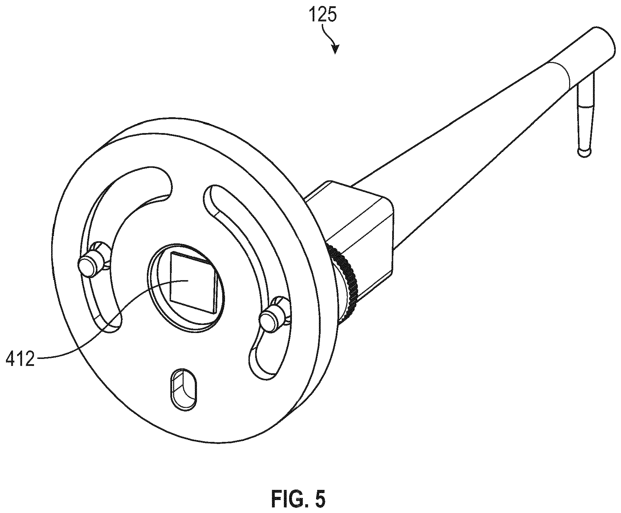

FIG. 5 shows an example the surgical instrument 125 with a data storage device 412 that can be utilized with the various systems previously discussed. The data storage device 412 can be the near field storage device (e.g., RFID, NFC, etc.) or micro-chip device configured for use with an electrical contact reading device. The data storage device 412 can be embedded or mounted to the surgical instrument 125 in any location and need not be optically visible.

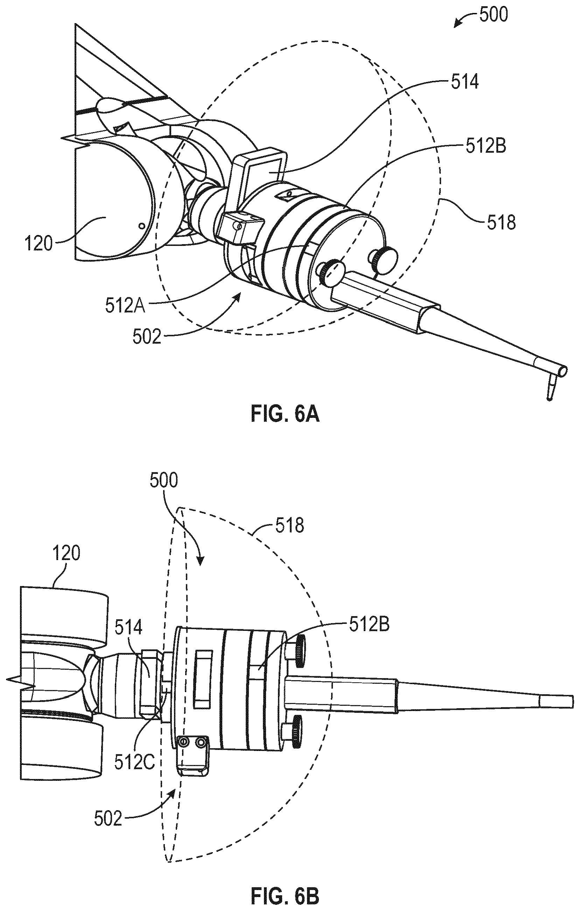

FIGS. 6A and 6B illustrate an example of an instrument holder 500 (specially an end effector 502) having a data reading device 514 mounted thereto. The data reading device 514 can be used with the identification system discussed previously. The data reading device 514 a near field reading device (e.g., RFID, NFC, etc.) or an OCR device, for example. The data reading device 514 can read data from a communication unit that is part of a tag of the RFID or NFC device or can read data optically that is part of a visual identifier. The data reading device 514 can send the read data to the computing system 140 (FIG. 1) or other processing circuitry including the cloud via the communications network. The data reading device 514 can be configured to read data from one or more of the data storage devices 512A, 512B, and/or 512C as shown in FIGS. 6A and 6B. The data reading device 514 can be mounted to the end effector 502 adjacent the robotic arm 120. The data reading device 514 can have a range or field 518 sufficient such that the data storage devices 512A, 512B, and/or 512C mounted to the robotic arm 120 fall within the range or field so as to be identifiable for reading and writing data thereto.

According to some examples, the data storage devices 512A, 512B, and/or 512C can have identification data that is linked or otherwise associated according to criteria of the identification system 190 and/or the computing system 140 (FIG. 1). According to this criteria, each of the data storage devices 512A, 512B, and/or 512C must present identification (including authentication, validation, use authorization, part number or other identifier) that matches or is otherwise linked to or validated for use with others of the data storage devices 512A, 512B, and/or 512C. In this manner it can be ensured that only a proper instrument for the stage of the surgery, proper accessory(s) associated with that instrument and/or a proper end effector associated with that instrument and/or accessory(s) can satisfy all identification data criteria in order to be used during the step of the surgery.

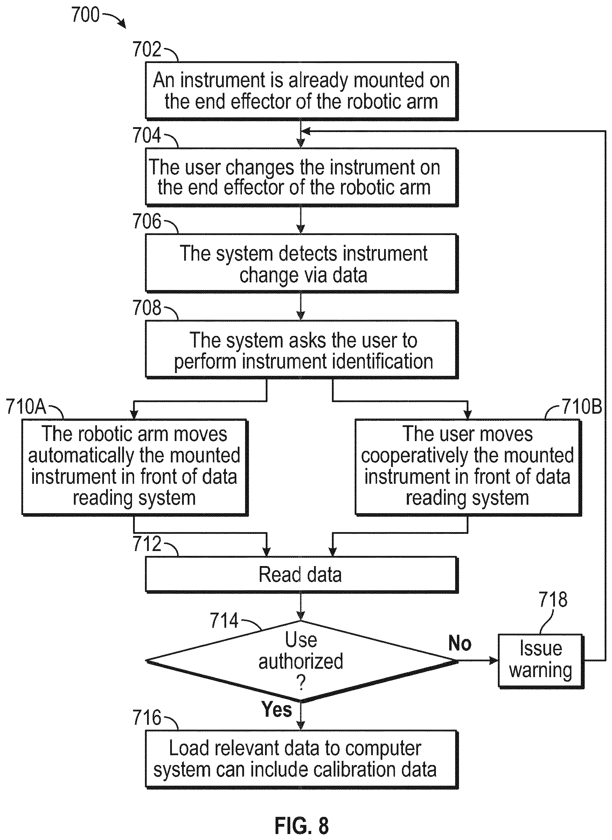

FIGS. 7-10 show various methods of validating one or more of an instrument or accessory for use during a robotically performed surgical procedure. The method 600 of FIG. 7 and the method 700 of FIG. 8 can include a data reading device or system that is cloud based or embedded onto the robot stand. According to these methods 600 and 700, to identify the instrument/accessory(s) mounted on the end effector of the robotic arm, the robotic arm can move automatically or cooperatively the mounted instrument/accessory(s) inside a range or field of view of the data reading device. The methods 600 and 700 can ensure that the instrument and/or accessory(s) is correct for that stage of the surgery and is correctly oriented and/or positioned in the right area in order to allow data reading by the data reading device.

The method 600 of FIG. 7 provides that no instrument and/or accessory(s) are mounted to an end effector of a robotic arm 602. Optionally, the method 600 or system can detect no instrument is mounted via data at step 604. This data can be obtained from a force sensor or from reading a data storage device, for example. The method 600 or system can prompt a user to mount an instrument and/or accessory(s) 606. The method 600 or system can detect a mounted instrument via data and can ask the user to perform instrument identification 608. This data can be obtained from the force sensor or from reading the data storage device, for example. To accomplish identification, the method 600 or system can have the robotic arm automatically move the mounted instrument with a data storage device into range of a data reading system or device 610A for data transfer. Alternatively, the user can move the robotic arm and instrument cooperatively to a position where the mounted instrument with a data storage device is in range of a data reading system or device for data transfer 610B. The method 600 or system can read the information (i.e. data) at step 612. The method 600 or system can determine if the information (i.e. data) from the transfer is correct (i.e. can the instrument, etc. be used?) at step 614. Such determination can include analysis of various criteria as previously discussed (e.g., Based upon the identification data is the instrument and/or accessory(s) the proper instrument for the stage in the surgery?; Based upon identification data are the instrument, the accessory(s) and/or the end effector the proper combination for use with one another?; Based upon the data are one or more of the instrument, the accessory(s) and/or the end effector mounted correctly?; Based upon the information data are one or more of the instrument, the accessory(s) and/or the end effector authentic?). If the information is determined to be correct, the method 600 or system can load relevant data in a computer system such as a robot embedded computer 616. Such data can include calibration data that can be stored on data storage devices as previously discussed. If the information is deemed incorrect for various reasons (i.e., invalid identification, unauthorized identification, counterfeit device, improper combination of identifiers, improper mounting, etc.) the method 600 or system can issue a warning 618 and prompt the user to mount another instrument, accessory(s) and/or end effector. The warning 618 can clarify the nature of the incorrect information (specify invalid identification, unauthorized identification, counterfeit device, improper combination of identifiers, improper mounting, etc.).

FIG. 8 shows a method 700 similar to that of the method 600 of FIG. 7. However, the method 700 differs in that the method 700 or system begins with a different surgical instrument than appropriate for the stage of the surgery already mounted to the end effector of the robotic arm 702. The method 700 or system can prompt the user to change the surgical instrument and/or accessory(s) on the end effector of the robotic arm 704. The method 700 or system can detect the surgical instrument and/or accessory(s) change via data at step 706. This data can be obtained from a force sensor or from reading a data storage device, for example. The method 700 or system can ask the user to perform instrument identification 708. To accomplish identification, the method 700 or system can have the robotic arm automatically move the mounted instrument with a data storage device into range of a data reading system or device 710A for data transfer. Alternatively, the user can move the robotic arm and instrument cooperatively to a position where the mounted instrument with a data storage device is in range of a data reading system or device for data transfer 710B. The method 700 or system can read the information (i.e. data) at step 712. The method 700 or system can determine if the information (i.e. data) from the transfer is correct at step 714. Such determination can include analysis of various criteria as previously discussed (e.g., Based upon the identification data is the instrument and/or accessory(s) the proper instrument for the stage in the surgery?; Based upon identification data are the instrument, the accessory(s) and/or the end effector the proper combination for use with one another?; Based upon the data are one or more of the instrument, the accessory(s) and/or the end effector mounted correctly?; Based upon the information data are one or more of the instrument, the accessory(s) and/or the end effector authentic?). If the information is determined to be correct, the method 700 or system can load relevant data in a computer system such as a robot embedded computer 716. If the information is deemed incorrect for various reasons (i.e., invalid identification, unauthorized identification, counterfeit device, improper combination of identifiers, improper mounting, etc.) the method 700 or system can issue a warning 718 and prompt the user to mount another instrument, accessory(s) and/or end effector. The warning 718 can clarify the nature of the incorrect information (specify invalid identification, unauthorized identification, counterfeit device, improper combination of identifiers, improper mounting, etc.).

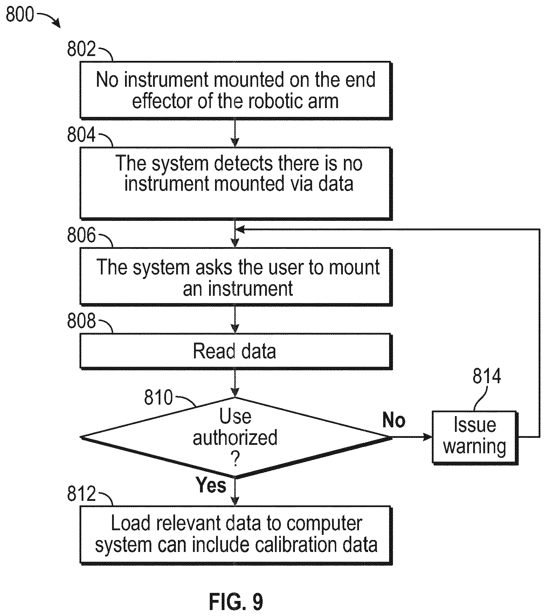

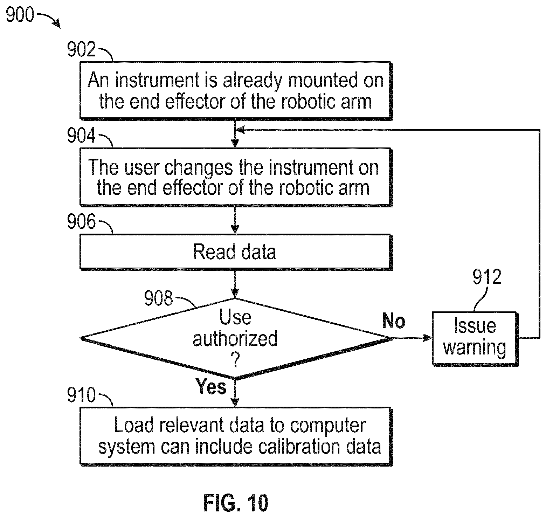

FIGS. 9 and 10 differ from the methods 600 and 700 of FIGS. 7 and 8 in that the data reading device or system can be embedded in or coupled onto the end effector of the robotic arm such as previously illustrated in FIGS. 6A and 6B. In these methods or systems, information encoded on the instrument and/or accessory(s) can be directly read by the data reading device when the instrument and/or accessory(s) is mounted. In FIGS. 9 and 10, no movement of the robotic arm is then required.

FIG. 9 shows a method 800 or system similar to that of FIG. 7. The method 800 or system of FIG. 9 provides that no instrument and/or accessory(s) are mounted to an end effector of a robotic arm 802. Optionally, the method 800 or system can detect no instrument is mounted via data at step 804. This data can be obtained from a force sensor or from reading a data storage device, for example. The method 800 or system can prompt a user to mount an instrument and/or accessory(s) 806. Optionally, the method 800 or system can detect a mounted instrument via data. This data can be obtained from the force sensor or from reading the data storage device, for example. The method 800 or system can read data from one or more data recording devices 808. The method 800 or system can determine if the information (i.e. data) from the transfer is correct at step 810. Such determination can include analysis of various criteria as previously discussed (e.g., Based upon the identification data is the instrument and/or accessory(s) the proper instrument for the stage in the surgery?; Based upon identification data are the instrument, the accessory(s) and/or the end effector the proper combination for use with one another?; Based upon the data are one or more of the instrument, the accessory(s) and/or the end effector mounted correctly?; Based upon the information data are one or more of the instrument, the accessory(s) and/or the end effector authentic?). If the information is determined to be correct, the method 800 or system can load relevant data in a computer system such as a robot embedded computer 812. If the information is deemed incorrect for various reasons (i.e., invalid identification, unauthorized identification, counterfeit device, improper combination of identifiers, improper mounting, etc.) the method 800 or system can issue a warning 814 and prompt the user to mount another instrument, accessory(s) and/or end effector. The warning 814 can clarify the nature of the incorrect information (specify invalid identification, unauthorized identification, counterfeit device, improper combination of identifiers, improper mounting, etc.).

FIG. 10 shows a method 900 or system begins with a different surgical instrument than appropriate for the stage of the surgery already mounted to the end effector of the robotic arm 902. The method 900 or system can prompt the user to change the surgical instrument and/or accessory(s) on the end effector of the robotic arm 904. The method 900 or system can read the information (i.e. data) 906. The method 900 or system can determine if the information (i.e. data) from the transfer is correct 908. Such determination can include analysis of various criteria as previously discussed (e.g., Based upon the identification data is the instrument and/or accessory(s) the proper instrument for the stage in the surgery?; Based upon identification data are the instrument, the accessory(s) and/or the end effector the proper combination for use with one another?; Based upon the data are one or more of the instrument, the accessory(s) and/or the end effector mounted correctly?; Based upon the information data are one or more of the instrument, the accessory(s) and/or the end effector authentic?). If the information is determined to be correct, the method 900 or system can load relevant data in a computer system such as a robot embedded computer 910. If the information is deemed incorrect for various reasons (i.e., invalid identification, unauthorized identification, counterfeit device, improper combination of identifiers, improper mounting, etc.) the method 900 or system can issue a warning 912 and can prompt the user to mount another instrument, accessory(s) and/or end effector.

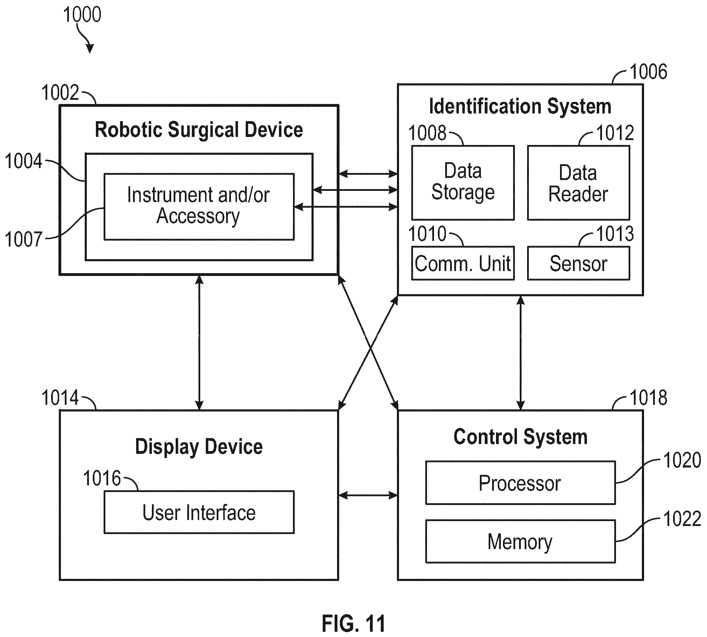

FIG. 11 illustrates system 1000 for performing techniques described herein including validating one or more of an instrument or accessory for use during a robotically performed surgical procedure, in accordance with some embodiments. The system 1000 can include robotic surgical device 1002 coupled to adjustable instrument holder 1004 (e.g., instrument holder 200 that includes the end effector 202), which may interact with an identification system 1006. The indentation system 1006 can interact with the instrument and/or accessory(s) 1007 as previously discussed to read and/or write data as desired for various purposes including identifying that a proper surgical instrument and/or accessory 1007 is mounted to the instrument holder 1004. The identification system 1006 can include data storage device(s) 1008, optionally a communication device 1010 (e.g. antenna, indicia, port, etc.), a data reader 1012 and optionally one or more sensors 1013 (e.g. force, acceleration, vector sensor, or the like). The system 1000 can include display device 1014, which can be used to display user interface 1016. The system 1000 can include a control system 1018 (e.g., a robotic controller), including a processor 1020 and memory 1022. In an example, control system 1018 can be coupled to one or more of robotic surgical device 1002, the identification system 1006, and/or the display device 1014.

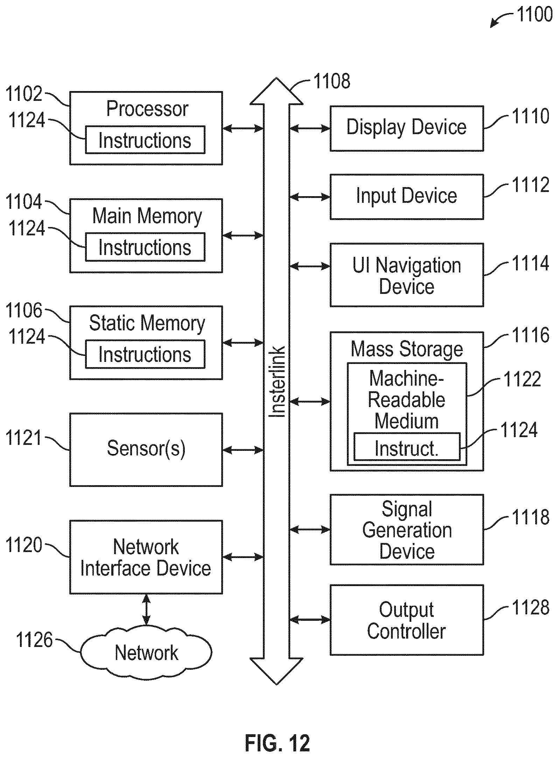

FIG. 12 illustrates a block diagram of an example machine 1100 upon which any one or more of the techniques discussed herein may perform in accordance with some embodiments. In alternative embodiments, machine 1100 may operate as a standalone device or may be connected (e.g., networked) to other machines. In a networked deployment, machine 1100 may operate in the capacity of a server machine, a client machine, or both in server-client network environments. In an example, machine 1100 may act as a peer machine in peer-to-peer (P2P) (or other distributed) network environment. Machine 1100 may be a personal computer (PC), a tablet PC, a set-top box (STB), a personal digital assistant (PDA), a mobile telephone, a web appliance, a network router, switch or bridge, or any machine capable of executing instructions (sequential or otherwise) that specify actions to be taken by that machine. Further, while only a single machine is illustrated, the term "machine" shall also be taken to include any collection of machines that individually or jointly execute a set (or multiple sets) of instructions to perform any one or more of the methodologies discussed herein, such as cloud computing, software as a service (SaaS), other computer cluster configurations.

Machine (e.g., computer system) 1100 may include hardware processor 1102 (e.g., a central processing unit (CPU), a graphics processing unit (GPU), a hardware processor core, or any combination thereof), main memory 1104 and static memory 1106, some or all of which may communicate with each other via interlink (e.g., bus) 1108. Machine 1100 may further include display unit 1110, alphanumeric input device 1112 (e.g., a keyboard), and user interface (UI) navigation device 1114 (e.g., a mouse). In an example, display unit 1110, input device 1112 and UI navigation device 1114 may be a touch screen display. Machine 1100 may additionally include storage device (e.g., drive unit) 1116, signal generation device 1118 (e.g., a speaker), network interface device 1120, and one or more sensors 1121, such as a global positioning system (GPS) sensor, compass, accelerometer, or other sensor. Machine 1100 may include output controller 1128, such as a serial (e.g., Universal Serial Bus (USB), parallel, or other wired or wireless (e.g., infrared (IR), near field communication (NFC), etc.) connection to communicate or control one or more peripheral devices (e.g., a printer, card reader, etc.).

Storage device 1116 may include machine readable medium 1122 on which is stored one or more sets of data structures or instructions 1124 (e.g., software) embodying or utilized by any one or more of the techniques or functions described herein. Instructions 1124 may also reside, completely or at least partially, within main memory 1104, within static memory 1106, or within hardware processor 1102 during execution thereof by machine 1100. In an example, one or any combination of hardware processor 1102, main memory 1104, static memory 1106, or storage device 1116 may constitute machine readable media.

While machine readable medium 1122 is illustrated as a single medium, the term "machine readable medium" may include a single medium or multiple media (e.g., a centralized or distributed database, and/or associated caches and servers) configured to store the one or more instructions 1124. The term "machine readable medium" may include any medium that is capable of storing, encoding, or carrying instructions for execution by machine 1100 and that cause machine 1100 to perform any one or more of the techniques of the present disclosure, or that is capable of storing, encoding or carrying data structures used by or associated with such instructions. Non-limiting machine readable medium examples may include solid-state memories, and optical and magnetic media.

Instructions 1124 may further be transmitted or received over communications network 1126 using a transmission medium via network interface device 1120 utilizing any one of a number of transfer protocols (e.g., frame relay, internet protocol (IP), transmission control protocol (TCP), user datagram protocol (UDP), hypertext transfer protocol (HTTP), etc.). Example communication networks may include a local area network (LAN), a wide area network (WAN), a packet data network (e.g., the Internet), mobile telephone networks (e.g., cellular networks), Plain Old Telephone (POTS) networks, and wireless data networks (e.g., Institute of Electrical and Electronics Engineers (IEEE) 802.11 family of standards known as Wi-Fi^{®}, IEEE 802.16 family of standards known as WiMax^{®}), IEEE 802.15.4 family of standards, peer-to-peer (P2P) networks, among others. In an example, network interface device 1120 may include one or more physical jacks (e.g., Ethernet, coaxial, or phone jacks) or one or more antennas to connect to communications network 1126. In an example, network interface device 1120 may include a plurality of antennas to wirelessly communicate using at least one of single-input multiple-output (SIMO), multiple-input multiple-output (MIMO), or multiple-input single-output (MISO) techniques. The term "transmission medium" shall be taken to include any intangible medium that is capable of storing, encoding or carrying instructions for execution by machine 1100, and includes digital or analog communications signals or other intangible medium to facilitate communication of such software.

The systems, devices and methods discussed in the present application can be useful in performing robotic-assisted surgical procedures that utilize robotic surgical arms that can be coupled to instrument holders used to precisely align trajectories of instruments relative to anatomy of a patient registered to the space of an operating room. The present disclosure describes adjustable instrument holders that can remain mounted to a robotic surgical arm throughout a surgical procedure. The adjustable instrument holders can be adjusted to hold instruments of different sizes, e.g., different diameters, without removing the instrument holder form the robotic arm. The adjustable instrument holders can be easily and quickly manipulated to remove a first instrument of a first size and insert a second instrument of a second size, thereby decreasing time for performing a surgical procedure. The adjustable instrument holders can include passages that have variable orifice sizes, e.g., variable diameters, formed by adjustable members, such as jaws or blades, that form adjustable jaws, chucks or diaphragms to align an instrument and hold an instrument along a trajectory. The adjustable instrument holders can include adjustment members that provide axial length along an axis of the trajectory to provide stability to the instrument. The adjustable instrument holders can additionally be easily and quickly assembled and disassembled for cleaning, sanitizing and sterilizing procedures. The scope of the invention should be determined with reference to the appended claims, along with the full scope of equivalents to which such claims are entitled.

## Claims

1. A robotic surgical system comprising:
a robotic arm connected to a base;
an end effector connected to a distal end of the robotic arm;
at least one instrument or accessory configured to mount to the end effector;
a data storage device attached to or positioned onboard the instrument or accessory;
a data reading device;
processing circuitry configured to:
receive a first data from the data reading device regarding the data storage device;
determine based upon the first data an identity of the at least one instrument or accessory; and
in response to such determination, load calibration data corresponding to the least one instrument or accessory or issue a warning regarding the at least one instrument or accessory;
a sensor, wherein the processing circuitry is configured to:
receive a second data regarding the sensor; and
determine based upon the second data at least a presence of the at least one instrument or accessory mounted on the end effector and
in response to the second data, the processing circuitry proceeds to determine based upon the first data the identity of the at least one instrument or accessory, wherein based upon the second data, the processing circuitry determines if the at least one instrument or accessory is installed properly.

2. The robotic surgical system of any one of claim 1, wherein the data storage device comprises one or more indicia affixed to or embedded **in** the at least one instrument or accessory, and wherein the data reading device comprises an optical device configured to perform image acquisition on the one or more indicia indicative of the first data.

3. The robotic surgical system of claim 1, wherein the data reading device is attached to or positioned onboard the accessory or the end effector.

4. The robotic surgical system of claim 3, wherein data reading device communicates with the data storage device to read and write information thereto.

5. The robotic surgical system of any one of claims 1-4, wherein the data storage device carries additional data including one or more of an authentication, a serial number, calibration data, usage data, sterilization data or operation data associated with the at least one instrument or accessory.

6. The robotic surgical system of any one claims 1-5, wherein the data storage device is attached to or positioned within the at least one instrument or accessory and comprises a Radio Frequency Identification (RFID) tag.

7. The robotic surgical system of any one of claims 1-5, wherein the data storage device is attached to or positioned within the at least one instrument or accessory and the data storage device and data reading device are configured to communicate through one of a wireless communication or an electric contact reading system.

8. The robotic surgical system of claim 1, wherein the data reading device is located at one or more of a remote cloud based location, onboard the base, onboard the end effector, or onboard the accessory.

9. The robotic surgical system of any one of claims 1-8, wherein the at least one instrument or accessory comprises one or more instruments and one or more accessories, and wherein based upon the first data the processing circuitry determines if the one or more accessories are configured for use with the one or more instruments and issues the warning if the one or more accessories are not configured for use with the one or more instruments.

10. The robotic surgical system of any one of claims 1-9, wherein the at least one instrument or accessory comprises one or more instruments and one or more accessories, and wherein based upon the first data the processing circuitry determines if the one or more instruments are authentic and issues the warning if the one or more instruments are counterfeit.

11. The robotic surgical system of any one of claims 1-10, wherein the at least one instrument or accessory comprises one or more instruments and one or more accessories, and wherein based upon the first data the processing circuitry determines if the one or more instruments are properly mounted on the one or more accessories and issues the warning if the one or more instruments are not properly mounted on the one or more accessories.

12. A computer implemented method of validating one or more of an instrument or accessory for use during a robotically performed surgical procedure, the method comprising:
coupling the one or more of the instrument or the accessory to an end effector of a robotic arm connected to a base;
communicating via a data reading device with a data storage system affixed to or embedded in the one or more of the instrument or the accessory;
identifying the one or more of the instrument or the accessory based upon data from the data storage system; and
based upon the identifying the one or more of the instrument or the accessory, loading a calibration associated with the one or more of the instrument or the accessory or issuing a warning regarding the one or more of the instrument or the accessory;
receive a second data from a sensor; and determine based upon the second data at least a presence of the at least one instrument or accessory mounted on the end effector and
in response to the second data, determine based upon the first data the identity of the at least one instrument or accessory,
wherein based upon the second data, determine if the at least one instrument or accessory is installed properly.

13. The method of claim 12, further comprising:
receiving sensor data from a sensor;
determining from the sensor data a presence of the one or more of the instrument or the accessory mounted on the end effector; and
in response to the determining from the sensor data the presence of the one or more of the instrument or the accessory mounted on the end effector, identifying the one or more of the instrument or the accessory based upon the data from the data storage system.

## Patentansprüche

1. Chirurgisches Robotersystem, umfassend:
einen Roboterarm, der mit einer Basis verbunden ist;
einen Endeffektor, der mit einem distalen Ende des Roboterarms verbunden ist;
mindestens ein Instrument oder Zubehörteil, das so konfiguriert ist, dass es an dem Endeffektor montiert wird;
eine Datenspeichervorrichtung, die an dem Instrument oder Zubehörteil angebracht oder an Bord von diesem positioniert ist;
eine Datenlesevorrichtung;
Verarbeitungsschaltungsanordnung, die so konfiguriert ist, dass sie:
erste Daten von der Datenlesevorrichtung in Bezug auf die Datenspeichervorrichtung empfängt;
auf der Grundlage der ersten Daten eine Identität des mindestens einen Instruments oder Zubehörteils bestimmt; und
als Reaktion auf eine solche Bestimmung Lastkalibrierungsdaten, die dem mindestens einen Instrument oder Zubehörteil entsprechen, oder eine Warnung bezüglich des mindestens einen Instruments oder Zubehörteils ausgibt;
einen Sensor, wobei die Verarbeitungsschaltungsanordnung so konfiguriert ist, dass sie:
zweite Daten bezüglich des Sensors empfängt; und
auf der Grundlage der zweiten Daten mindestens ein Vorhandensein des mindestens einen am Endeffektor montierten Instruments oder Zubehörteils bestimmt, und
als Reaktion auf die zweiten Daten die Verarbeitungsschaltungsanordnung fortfährt, auf der Grundlage der ersten Daten die Identität des mindestens einen Instruments oder Zubehörteils zu bestimmen, wobei die Verarbeitungsschaltungsanordnung auf der Grundlage der zweiten Daten bestimmt, ob das mindestens eine Instrument oder Zubehörteil ordnungsgemäß installiert ist.

2. Chirurgisches Robotersystem nach einem der Anspruch 1, wobei die Datenspeichervorrichtung eine oder mehrere Markierungen umfasst, die an dem mindestens einen Instrument oder Zubehörteil befestigt oder darin eingebettet sind, und wobei die Datenlesevorrichtung eine optische Vorrichtung umfasst, die so konfiguriert ist, dass sie eine Bilderfassung an der einen oder den mehreren Markierungen durchführt, die die ersten Daten anzeigen.

3. Chirurgisches Robotersystem nach Anspruch 1, wobei die Datenlesevorrichtung an dem Zubehörteil oder dem Endeffektor angebracht oder an Bord von diesem positioniert ist.

4. Chirurgisches Robotersystem nach Anspruch 3, wobei die Datenlesevorrichtung mit der Datenspeichervorrichtung kommuniziert, um Informationen darin zu lesen und darauf zu schreiben.

5. Chirurgisches Robotersystem nach einem der Ansprüche 1-4, wobei die Datenspeichervorrichtung zusätzliche Daten trägt, einschließlich eines oder mehrerer von einer Authentifizierung, einer Seriennummer, Kalibrierungsdaten, Nutzungsdaten, Sterilisationsdaten oder Betriebsdaten, die mit dem mindestens einen Instrument oder Zubehörteil assoziiert sind.

6. Chirurgisches Robotersystem nach einem der Ansprüche 1-5, wobei die Datenspeichervorrichtung an dem mindestens einen Instrument oder Zubehörteil angebracht oder darin positioniert ist und ein Radiofrequenz-Identifikations (RFID) -Tag umfasst.

7. Chirurgisches Robotersystem nach einem der Ansprüche 1-5, wobei die Datenspeichervorrichtung an dem mindestens einen Instrument oder Zubehörteil angebracht oder darin positioniert ist und die Datenspeichervorrichtung und die Datenlesevorrichtung so konfiguriert sind, dass sie über eines von einer drahtlosen Kommunikation oder einem elektrischen Kontaktlesesystem kommunizieren.

8. Chirurgisches Robotersystem nach Anspruch 1, wobei sich die Datenlesevorrichtung an einem oder mehreren von einem entfernten cloudbasierten Standort, an Bord der Basis, an Bord des Endeffektors oder an Bord des Zubehörteils befindet.

9. Chirurgisches Robotersystem nach einem der Ansprüche 1-8, wobei das mindestens eine Instrument oder Zubehörteil ein oder mehrere Instrumente und ein oder mehrere Zubehörteile umfasst, und wobei die Verarbeitungsschaltungsanordnung auf Grundlage der ersten Daten bestimmt, ob das eine oder die mehreren Zubehörteile für die Verwendung mit dem einen oder den mehreren Instrumenten konfiguriert sind, und die Warnung ausgibt, wenn das eine oder die mehreren Zubehörteile nicht für die Verwendung mit dem einen oder den mehreren Instrumenten konfiguriert sind.

10. Chirurgisches Robotersystem nach einem der Ansprüche 1-9, wobei das mindestens eine Instrument oder Zubehörteil ein oder mehrere Instrumente und ein oder mehrere Zubehörteile umfasst, und wobei die Verarbeitungsschaltungsanordnung auf Grundlage der ersten Daten bestimmt, ob das eine oder die mehreren Instrumente authentisch sind, und die Warnung ausgibt, wenn das eine oder die mehreren Instrumente gefälscht sind.

11. Chirurgisches Robotersystem nach einem der Ansprüche 1-10, wobei das mindestens eine Instrument oder Zubehörteil ein oder mehrere Instrumente und ein oder mehrere Zubehörteile umfasst, und wobei die Verarbeitungsschaltungsanordnung auf Grundlage der ersten Daten bestimmt, ob das eine oder die mehreren Instrumente richtig an dem einen oder den mehreren Zubehörteilen montiert sind, und die Warnung ausgibt, wenn das eine oder die mehreren Instrumente nicht richtig an dem einen oder den mehreren Zubehörteilen montiert sind.

12. Computerimplementiertes Verfahren des Validierens eines oder mehrerer von einem Instrument oder Zubehörteil zur Verwendung während eines robotisch gestützten chirurgischen Eingriffs, wobei das Verfahren umfasst:
Koppeln des einen oder der mehreren des Instruments oder des Zubehörteils an einen Endeffektor eines mit einer Basis verbundenen Roboterarms;
Kommunizieren über eine Datenlesevorrichtung mit einem Datenspeichersystem, das an dem einen oder den mehreren des Instruments oder des Zubehörteils befestigt oder darin eingebettet ist;
Identifizieren des einen oder der mehreren des Instruments oder des Zubehörteils, auf der Grundlage von Daten aus dem Datenspeichersystem; und
auf der Grundlage des Identifizierens des einen oder der mehreren des Instruments oder des Zubehörteils, Laden einer Kalibrierung, die dem einen oder den mehreren des Instruments oder des Zubehörteils zugeordnet ist, oder Ausgeben einer Warnung bezüglich des einen oder der mehreren des Instruments oder des Zubehörteils;
Empfangen von zweiten Daten von einem Sensor; und Bestimmen, auf der Grundlage der zweiten Daten, mindestens eines Vorhandenseins des mindestens einen Instruments oder Zubehörteils, das an dem Endeffektor montiert ist, und,
als Reaktion auf die zweiten Daten, Bestimmen, auf der Grundlage der ersten Daten, der Identität des mindestens einen Instruments oder Zubehörteils,
wobei auf der Grundlage der zweiten Daten bestimmt wird, ob das mindestens eine Instrument oder Zubehörteil richtig installiert ist.

13. Verfahren nach Anspruch 12, ferner umfassend:
Empfangen von Sensordaten von einem Sensor;
Bestimmen einer Anwesenheit des einen oder der mehreren des Instruments oder des Zubehörteils, das an dem Endeffektor montiert ist, aus den Sensordaten; und
als Reaktion auf das Bestimmen des Vorhandenseins des einen oder der mehreren des Instruments oder des Zubehörteils, das an dem Endeffektor montiert ist, aus den Sensordaten, Identifizieren des einen oder der mehreren des Instruments oder des Zubehörteils auf Grundlage der Daten aus dem Datenspeichersystem.

## Revendications

1. Système chirurgical robotisé comprenant :
un bras robotisé relié à une base ;
un effecteur d'extrémité connecté à une extrémité distale du bras robotisé ;
au moins un instrument ou accessoire configuré pour s'assembler à l'effecteur d'extrémité ;
un dispositif de stockage de données attaché à ou positionné embarqué dans l'instrument ou l'accessoire ;
un dispositif de lecture de données ;
des circuits de traitement configurés pour :
recevoir une première donnée en provenance du dispositif de lecture de données concernant le dispositif de stockage de données ;
déterminer, sur la base de la première donnée, une identité de l'au moins un instrument ou accessoire ; et
en réponse à une telle détermination, charger les données d'étalonnage correspondant à l'au moins un instrument ou accessoire ou émettre un avertissement concernant l'au moins un instrument ou accessoire ;
un capteur, dans lequel les circuits de traitement sont configurés pour :
recevoir une deuxième donnée concernant le capteur ; et
déterminer sur la base de la deuxième donnée au moins une présence de l'au moins un instrument ou accessoire monté sur l'effecteur d'extrémité et
en réponse à la deuxième donnée, les circuits de traitement procède à la détermination, sur la base de la première donnée, de l'identité de l'au moins un instrument ou accessoire, dans lequel, sur la base de la deuxième donnée, les circuits de traitement déterminent si l'au moins un instrument ou accessoire est installé correctement.

2. Système chirurgical robotisé selon l'une quelconque de la revendication 1, dans lequel le dispositif de stockage de données comprend un ou plusieurs indices fixés à ou intégrés dans l'au moins un instrument ou accessoire, et dans lequel le dispositif de lecture de données comprend un dispositif optique configuré pour effectuer une acquisition d'image sur l'un ou plusieurs indices indicatifs de la première donnée.

3. Système chirurgical robotisé selon la revendication 1, dans lequel le dispositif de lecture de données est attaché à ou positionné embarqué dans l'accessoire ou l'effecteur d'extrémité.

4. Système chirurgical robotisé selon la revendication 3, dans lequel le dispositif de lecture de données communique avec le dispositif de stockage de données pour lire et écrire des informations sur celui-ci.

5. Système chirurgical robotisé selon l'une quelconque des revendications 1 à 4, dans lequel le dispositif de stockage de données porte des données supplémentaires comprenant une ou plusieurs données parmi une authentification, un numéro de série, des données d'étalonnage, des données d'utilisation, des données de stérilisation ou des données de fonctionnement associées à l'au moins un instrument ou accessoire.

6. Système chirurgical robotisé selon l'une quelconque des revendications 1 à 5, dans lequel le dispositif de stockage de données est attaché à ou positionné à l'intérieur de l'au moins un instrument ou accessoire et comprend une étiquette d'identification par radiofréquence (RFID).

7. Système chirurgical robotisé selon l'une quelconque des revendications 1 à 5, dans lequel le dispositif de stockage de données est attaché à ou positionné à l'intérieur de l'au moins un instrument ou accessoire et le dispositif de stockage de données et le dispositif de lecture de données sont configurés pour communiquer via l'un d'une communication sans fil ou d'un système de lecture de contact électrique.

8. Système chirurgical robotisé selon la revendication 1, dans lequel le dispositif de lecture de données est situé à un ou plusieurs endroits parmi un emplacement basé sur un nuage distant, embarqué dans la base, embarqué dans l'effecteur d'extrémité ou embarqué dans l'accessoire.

9. Système chirurgical robotisé selon l'une quelconque des revendications 1 à 8, dans lequel l'au moins un instrument ou accessoire comprend un ou plusieurs instruments et un ou plusieurs accessoires, et dans lequel, sur la base de la première donnée, les circuits de traitement déterminent si l'un ou plusieurs accessoires sont configurés pour une utilisation avec l'un ou plusieurs instruments et émettent l'avertissement si l'un ou plusieurs accessoires ne sont pas configurés pour une utilisation avec l'un ou plusieurs instruments.

10. Système chirurgical robotisé selon l'une quelconque des revendications 1 à 9, dans lequel l'au moins un instrument ou accessoire comprend un ou plusieurs instruments et un ou plusieurs accessoires, et dans lequel, sur la base de la première donnée, les circuits de traitement déterminent si l'un ou plusieurs instruments sont authentiques et émettent l'avertissement si l'un ou plusieurs instruments sont contrefaits.

11. Système chirurgical robotisé selon l'une quelconque des revendications 1 à 10, dans lequel l'au moins un instrument ou accessoire comprend un ou plusieurs instruments et un ou plusieurs accessoires, et dans lequel, sur la base de la première donnée, les circuits de traitement déterminent si l'un ou plusieurs instruments sont correctement montés sur l'un ou plusieurs accessoires et émettent l'avertissement si l'un ou plusieurs instruments ne sont pas correctement montés sur l'un ou plusieurs accessoires.

12. Procédé mis en œuvre par ordinateur de validation d'un ou plusieurs instruments ou accessoires à utiliser pendant une procédure chirurgicale exécutée de manière robotisée, le procédé comprenant :
le couplage de l'un ou plusieurs de l'instrument ou de l'accessoire à un effecteur d'extrémité d'un bras robotisé relié à une base ;
la communication via un dispositif de lecture de données avec un système de stockage de données fixé à ou embarqué dans l'un ou plusieurs de l'instrument ou l'accessoire ;
l'identification de l'un ou plusieurs de l'instrument ou l'accessoire sur la base des données provenant du système de stockage de données ; et
sur la base de l'identification de l'un ou plusieurs de l'instrument ou l'accessoire, le chargement d'un étalonnage associé à l'un ou plusieurs de l'instrument ou l'accessoire ou l'émission d'un avertissement concernant l'un ou plusieurs de l'instrument ou l'accessoire ;
la réception d'une deuxième donnée en provenance d'un capteur ; et la détermination sur la base de la deuxième donnée d'au moins une présence de l'au moins un instrument ou accessoire monté sur l'effecteur d'extrémité et,
en réponse à la deuxième donnée, la détermination sur la base de la première donnée de l'identité de l'au moins un instrument ou accessoire,
dans lequel, sur la base de la deuxième donnée, la détermination si l'au moins un instrument ou accessoire est installé correctement.

13. Procédé selon la revendication 12, comprenant en outre :
la réception de données de capteur en provenance d'un capteur ;
la détermination à partir des données de capteur d'une présence de l'un ou plusieurs de l'instrument ou l'accessoire monté sur l'effecteur d'extrémité ; et
en réponse à la détermination à partir des données du capteur de la présence de l'un ou plusieurs de l'instrument ou l'accessoire monté sur l'effecteur d'extrémité,
l'identification de l'un ou plusieurs de l'instrument ou l'accessoire sur la base des données provenant du système de stockage de données.
